Europäisches Patentamt

European Patent Office

Office européen des brevets

(11) Veröffentlichungsnummer: **0 311 950 A2**

## (12) EUROPÄISCHE PATENTANMELDUNG

(21) Anmeldenummer: 88116775.3

(22) Anmeldetag: 10.10.88

(51) Int. Cl.⁴: **C07K 3/28** , //A61K35/16, A61K37/64,A61L2/00

(30) Priorität: 15.10.87 DE 3734923

(43) Veröffentlichungstag der Anmeldung:
19.04.89 Patentblatt 89/16

(84) Benannte Vertragsstaaten:
AT BE CH DE ES FR GB GR IT LI LU NL SE

(71) Anmelder: BIOTEST PHARMA GMBH
Landsteiner Strasse 5
D-6072 Dreieich(DE)

(72) Erfinder: Kotitschke,Ronald Dr., Dipl.-Chem.
Kleiststrasse 23
D-6072 Dreieich(DE)
Erfinder: Stephan,Wolfgang,Dr.Dipl.-Chem.
Philipp-Holzmann-Strasse 84
D-6072 Dreieich(DE)
Erfinder: Der weitere Erfinder hat auf seine
Nennung verzichtet

(74) Vertreter: Beil, Hans Chr., Dr. et al
Beil, Wolff und Beil, Rechtsanwälte
Adelonstrasse 58 Postfach 80 01 40
D-6230 Frankfurt am Main 80(DE)

(54) Verfahren zur Herstellung einer sterilen Plasmaproteinlösung, die Fibrinogen und den Gerinnungsfaktor XIII enthält.

(57) Die bisher angewendeten Verfahren zur Gewinnung von Fibrinogen und des Gerinnungsfaktors XIII aus Blutplasma führen entweder zu Produkten, die nicht hepatitissicher sind oder einen zu hohen Aufwand bezüglich der Sterilisation erfordern. Durch das neue Verfahren soll eine sterile und stabile Plasmaproteinlösung hergestellt werden, die beide Komponenten in einer erhöhten Konzentration enthält.

Ein mit Citrat stabilisiertes Blutplasma wird vor der Abtrennung der Gerinnungsfaktoren II, VII, IX und X mit Hilfe eines Anionenaustauschers durch Behandlung mit $\beta$-Propiolacton und Bestrahlung mit UV-Licht sterilisiert. Durch fraktionierte Fällung mit Ethanol werden nach Abtrennung der Begleitproteine Fibrinogen und der Gerinnungsfaktor XIII in konzentrierter Form erhalten.

Die sterile und stabile Plasmaproteinlösung ist besonders als Fibrinogenkleberkomponente geeignet.

# Verfahren zur Herstellung einer sterilen Plasmaproteinlösung, die Fibrinogen und den Gerinnungsfaktor XIII enthält

Die Erfindung betrifft ein Verfahren zur Herstellung einer sterilen Plasmaproteinlösung, die Fibrinogen und den Gerinnungsfaktor XIII enhält und sich besonders als Komponente eines Fibrinklebers eignet, aus einem mit Citrat stabilisierten humanen Blutplasma.

Aus der Literatur sind eine Anzahl verschiedener Verfahren der Fibrinogenherstellung aus Plasmen bekannt, von denen die meisten Varianten die von Cohn beschriebene Ethanolfällung anwenden (Journal of the American Society 72 (1950), 564-474). Eine bekannte Weiterentwicklung dieses Verfahrens ist von Blombäck u.a. beschrieben worden (Archiv für Kemi 10 (1956), Nr. 29, 415-443).

Blutplasmafraktionen haben ein hohes Risiko, infektiös zu sein. Dieses Risiko betrifft besonders die Übertragung der Hepatitis B und Hepatitis Non-A/Non-B und in jüngster Zeit der HIV (human immundeficiency virus).

Fibrinogen und die Cohn I-Fraktion gehören zu den sogenannten "high risk" Präparaten in bezug auf das Hepatitisrisiko (J.H.Hoofnagle u.a.: J.Lab.Clin.Med. 88 (1976) , 102; W. Doleschel u.a.: Wiener Klin-Wschr. 89 (1977), 383).

In den USA ist daher die klinische Anwendung von Fibrinogenpräparaten, die aus großen Plasmapools hergestellt werden, untersagt.

Da es durch diagnostische Maßnahmen nicht gelingt, hepatitissichere Blutprodukte aus Plasmapools herzustellen, sind verschiedene Verfahren zur Sterilisation von Blutbestandteilen entwickelt worden. Die Pasteurisierung (10h/60° C) wird erfolgreich für Albumin verwendet und ist durch die Verwendung von Stabilisatoren wie Aminosäuren und Mono- bzw. Oligosacchariden und Zuckeralkoholen seit einigen Jahren auch zur Sterilisation empfindlicher Plasmaproteine wie den Gerinnungsfaktoren II, VIII und XIII beschrieben worden (EP-B 0 018 561). Die Wirksamkeit der Pasteurisation in Gegenwart dieser Stabilisatoren ist noch nicht abzuschätzen und wird zur Zeit überprüft.

Die von LoGrippo beschriebene Methode der Kaltsterilisation besteht in der kombinierten Behandlung von Humanplasma mit β-Propiolacton und UV-Bestrahlung (G.A. LoGrippo und H.Hayashi: Henry Ford Hosp. Med. J. 21 (1973), 181; G.A. LoGrippo und F.W. Hartman: Bibl. Haematol.7 (1958), 225).

Die Sterilisation einer bereits gereinigten Fibrinogenfraktion mit β-Propiolacton/UV führt zu nicht mehr gerinnbaren Fibrinogenprodukten (R. Kotitschke und W.Stephan: Proteins and Related Subjects, Vol.28, Protides of Biological Fluids. Peeters H., 28.Colloquium 1980, Verlag Pergamon-Press, Oxford, New-York-Toronto-Paris, 333-337; H.Brunner u.a.:Verhandlungen der Deutsch.Gesellsch. für Inn.Med. 79, S.1130-1134 (1983)); W.Doleschel und W.Auerswald: Pharmacology 2 (1969), 1).

In der EP-B 0 014 333 ist ein Verfahren zur Herstellung der Plasmafraktionen, Fibrinogen, einem die Gerinnungsfaktoren II, VII, IX und X enthaltenden Prothrombinkomplex, Antithrombin III und einer Lösung von lagerstabilen Serumproteinen aus einem mit Citrat stabilisierten Blutplasma, das mit β-Propiolacton und UV-Bestrahlung behandelt wurde, beschrieben. Nach diesem Verfahren wird Fibrinogen aus einem Citratplasmapool, der β-Propiolacton-behandelt und UV-bestrahlt wurde, durch Adsorption an kolloidale Kieselsäure und anschließender Elution hergestellt. Dieses Fibrinogen ist als Komponente für einen Fibrinkleber ungeeignet, da ein derartiges Fibrinogen keinen Faktor XIII enthält. Damit ein Fibrinogenpräparat die Anforderungen erfüllt, die an ein Fibrinogen zu stellen sind, das für die Fibrinklebung geeignet ist, muß es mit einem F.XIII-Konzentrat angereichert werden.

Im biologischen Fibrinklebersystem werden durch den durch Ca-Ionen und Thrombin aktivierten Gerinnungsfaktor XIII (F.XIII) die einzelnen Fibrinpolymere quervernetzt, so daß ein unlösliches, festes und elastisches Fibringerinnsel entsteht.

(H.P.Spängler und F.Braun: Grundlagen der Fibrinklebung in der operativen Medizin, ed. H.P.Spängler und F.Braun: edition medizin. Weinheim; Deerfield Beach, Florida, Basel 1983).

Die Bedeutung des F.XIII-Gehaltes des Fibrinklebersys tems ist bei Hautklebungen in vivo nachgewiesen worden (H.P. Spängler u.a.: Wien. Klin. Wochenschr. 85, 827 (1973)).

Verfahren zur Herstellung von sterilen F.XIII-Konzentraten aus humanem Plasma sind sehr aufwendig, weshalb F.XIII-Konzentrate auch vorzugsweise aus humanen Placenten hergestellt werden (CA-PS 957 943). Die F.XIII-Konzentrat-Herstellung aus Plasma, aus dem das Kryopräzipitat entfernt worden ist, ist von Winkelman u.a. beschrieben worden (Thrombosis and Haemostasis 55, 3 (1986), 402-405).

Lösungen, die den menschlichen Blutgerinnungsfaktor XIII enthalten, müssen zur Vermeidung der Übertragung von Viren sterilisiert werden (EP-B 0 037 078).

Der vorliegenden Erfindung lag die Aufgabe zugrunde, aus humanem Plasma eine sterile und stabile Plasmaproteinlösung herzustellen, die sowohl den Gerinnungsfaktor XIII als auch Fibrinogen in gegenüber dem Plasma ankonzentrierter Menge enthält und besonders als Fibrinkleberkomponente geeignet ist.

Diese Aufgabe wird erfindungsgemäß dadurch gelöst, daß man das mit Citrat stabilisierte Blutplasma mit β-Propiolacton behandelt und mit ultraviolettem Licht bestrahlt, anschließend die Gerinnungsfaktoren II, VII, IX und X durch Adsorption an Proteine adsorbierende Anionenaustauscher abtrennt, nachfolgend eine Fällung mit Ethanol bis zu einer Endkonzentration der Lösung von 9 Vol.-% bei -3° C durchführt, den Niederschlag abzentrifugiert und in einem Citratpuffer bei einem pH-Wert von 6,35 und 37° C unter Rühren löst, den Proteinwert der Fibrinogenlösung mit Natriumcitratlösung auf 13,3 g/l einstellt und weitere Begleitproteine mit Ethanol, einem Glycin-Citrat-Puffer und Natriumcitratlösung ausfällt, den Niederschlag abzentrifugiert und aus dem Überstand das Fibrinogen und den Gerinnungsfaktor XIII durch Zugabe von Ethanol bis zu einer Endkonzentration der Lösung von 9 Vol.-% bei -3° C ausfällt, den Niederschlag bei einem pH-Wert von 6,35 und 37° C unter Rühren in einem Citratpuffer zu der gewünschten Fibrinogenkonzentration auflöst und die Lösung über eine Filterklärscheibe und ein Millipore Sterilfilter filtriert.

Die Behandlung des Blutplasmas mit β-Propiolacton erfolgt bei einem pH-Wert von 7,2 in einem Konzentrationsbereich von 0,2 bis 0,35 Vol.-%, bezogen auf das eingesetzte Plasma. Die UV-Dosis beträgt 4x20 Watt in 1 cm Abstand bei einer Schichtdicke des bestrahlten Plasmas von 0,15mm und einer Durchflußgeschwindigkeit von 20 l/h und 700 RPM (Rotationen des UV-Rohres pro Minute).

Wird die Fraktionierung eines β-Propiolacton/UV-behandelten Plasmas auf diese Weise ohne vorherige Abtrennung der Gerinnungsfaktoren II, VII, IX und X durchgeführt, erhält man eine Plasmafraktion, die das Fibrinogen und den Gerinnungsfaktor XIII in nicht stabiler Form enthält, d.h., die so hergestellten Produkte gelieren und gerinnen, so daß eine weitere Aufarbeitung nicht möglich ist.

Durch das erfindungsgemäße Verfahren wird aus einem mit Citrat stabilisierten humanen Blutplasma eine gerinnungsaktive, hepatitissichere und therapeutisch anwendbare Plasmaproteinlösung erhalten, die das Fibrinogen und den Gerinnungsfaktor XIII in gegenüber dem Plasma angereicherter Form enthält. Diese Plasmaproteinlösung ist besonders als Komponente eines Fibrinogenklebers geeignet.

Die nachstehende Tabelle zeigt die Eigenschaften von zwei nach dem erfindungsgemäßen Verfahren hergestellten Plasmaproteinlösungen, deren Proteinkonzentration in Abhängigkeit vom Verwendungszweck unterschiedlich eingestellt wurde.

Tabelle

| Protein (g/l | Fibrinogen (g/l) | Faktor XIII (E/ml) |
|---|---|---|
| 60 | 50 | 6 |
| 90 | 75 | 9 |

Die Erfindung wird durch das nachstehende Beispiel näher erläutert.

Ausführungsbeispiel

60 Blutplasmabeutel mit fresh frozen Citratplasma mit einem Gesamtgewicht von 37,2 kg wurden über Nacht bei +8° C belassen. Die Beutel wurden anschließend mit einem Skalpell unter dem Laminarflow geöffnet und das gefrorene Plasma in einem Kessel mit Heizmantel bei 37° C unter Rühren innerhalb 1h aufgetaut.

Der Plasmapool wurde innerhalb von ca. 45 Minuten auf ca. 10° C abgekühlt. 92,5 ml β-Propiolacton (0,25%) wurden unter Rühren tropfenweise zu dem Plasma gegeben. Nachdem der pH-Wert von 7,2 erreicht war, wurde dieser durch Zugabe von 1 N NaOH für 1h konstant bei Zimmertemperatur gehalten. Es wurden 1 1 1 N NaOH verbraucht. Das mit β-Propiolacton behandelte Plasma wurde mit einem Standard UV-Gerät einer UV-Dosis von 4x20 Watt in 1cm Abstand bei einer Schichtdicke des bestrahlten Plasmas von 0,15mm und einer Durchflußgeschwindigkeit von 20 l/h und 700 RPM (Rotation des UV-Rohres pro Minute) ausgesetzt.

Pro 1 kg Plasma wurden 1,5 g DEAE-Sephadex A 50 zur Adsorption der PPSB-Faktoren verwendet. Nach einer Rührzeit von 1 h setzte sich das Sephadex A-50 über Nacht ab. Am nächsten Tag wurde das über dem Sediment stehende Plasma mit Hilfe eines Steigrohres und einer Pumpe abgesaugt.

Der pH-Wert des Plasmas wurde auf 7,2 eingestellt und das Plasma auf 0° C abgekühlt. 96%iges Ethanol wurde unter Rühren bis zu einer Endkonzentration von 9% zugegeben. Die Temperatur wurde auf -3° C abgesenkt, die Fällzeit betrug 14h. Der Niederschlag wurde durch Zentrifugation bei einer Zentrifugentemperatur von -15°C und einer Zentrifugationszeit von 1 l/Min. bei 11.000 UpM mit einer WKF-Zentrifuge abgetrennt.

Die gefällte Plasmaproteinfraktion (350 g Niederschlag) wurde mit 1200 ml eines Citrat-Puffers bei pH 6,35 und +37°C unter Rühren innerhalb von 45 Minuten gelöst.

Zur Einstellung des Proteinwertes auf 13,3 g/l wurde eine Na-Citratlösng pH 6,35 (0,055 M Citrat) verwendet. Pro 1kg Fibrinogenlösung wurde für die Ausfällung von Begleitproteinen ein Glycin-Citrat-Puffer pH 6,35 und Ethanol in der folgenden Zusammensetzung verwendet: 260ml Ethanol, 240ml Glycin-Citrat-Puffer und 1500ml Citrat-Lösng. Nach einer Fällzeit von ca. 1h wurde der Niederschlag abzentrifugiert und aus dem Überstand das Fibrinogen und der Faktor XIII durch Zugabe von Ethanol bis zu einer Endkonzentration von 9% bei -3°C ausgefällt. Der Niederschlag wurde mit einem Lösungspuffer bei +37°C unter Rühren zu der gewünschten Fibrinogenkonzentration aufgelöst.

Die Fibrinogenlösung wurde zunächst über eine Filterklärscheibe und anschließend über ein Millipore-Sterilfilter filtriert. Die sterilfiltrierte Lösung wurde bis zur weiteren Verwendung bei -80°C tiefgefroren gelagert.

Nachfolgend ist das erfindungsgemäße Verfahren schematisch dargestellt:

**Schematische Darstellung der Herstellung der sterilen
Plasmaproteinlösung (Fibrinogen und F.XIII)**

```
                ┌─────────────────────┐
                │   Citratplasma      │
                └─────────────────────┘
                           │
                ┌─────────────────────┐
                │ Sterilisation mit ß-PL/UV │
                └─────────────────────┘
                           │
                ┌─────────────────────────────┐
                │ Adsorption an Anionenaustauscher │
                │ für Proteine                │
                └─────────────────────────────┘
                           │
┌─────────────┐            │
│ Niederschlag│────────────┤
└─────────────┘
      │
┌─────────────┐
│    PPSB     │
└─────────────┘
                           │
                ┌─────────────────────┐
                │   Überstand         │
                └─────────────────────┘
                           │
                ┌─────────────────────┐
                │  Ethanolfällung     │
                └─────────────────────┘
                           │
                ┌─────────────────────┐
                │ Niederschlag lösen  │
                └─────────────────────┘
                           │
                ┌─────────────────────────┐
                │ Ethanol/Glycin-Fällung  │
                └─────────────────────────┘
                           │
                ┌─────────────────────┐
                │   Überstand         │
                └─────────────────────┘
                           │
                ┌─────────────────────────┐
                │ Fibrinogen-F. XIII-Fällung │
                └─────────────────────────┘
                           │
                ┌─────────────────────┐
                │ Niederschlag lösen  │
                └─────────────────────┘
                           │
                ┌─────────────────────┐
                │  Sterilfiltration   │
                └─────────────────────┘
```

**Ansprüche**

1. Verfahren zur Herstellung einer sterilen und stabilen Plasmaproteinlösung, die Fibrinogen und den Gerinnungsfaktor XIII enthält, aus einem mit Citrat stabilisierten humanen Blutplasma, aus dem die Gerinnungsfaktoren II, VII, IX und X durch Adsorption an Proteine adsorbierende Anionenaustauscher abgetrennt werden und die Abtrennung weiterer Begleitproteine durch fraktionierte Fällung mit Ethanol erfolgt, dadurch gekennzeichnet, daß man das mit Citrat stabilisierte Blutplasma mit β-Propiolacton behandelt und mit ultraviolettem Licht bestrahlt, anschließend die Gerinnungsfaktoren II, VII, IX und X durch Adsorption an Proteine adsorbierende Anionenaustauscher abtrennt, nachfolgend eine Fällung mit Ethanol bis zu einer Endkonzentration der Lösung von 9 Vol.-% bei -3°C durchführt, den Niederschlag abzentrifugiert und in einem Citratpuffer bei einem pH-Wert von 6,35 und 37°C unter Rühren löst, den Proteinwert der Fibrinogenlösung mit Natriumcitratlösung auf 13,3 g/l einstellt und weitere Begleitproteine mit Ethanol, einem Glycin-Citrat-Puffer und Natriumcitratlösung ausfällt, den Niederschlag abzentrifugiert und aus dem Überstand das Fibrinogen und den Gerinnungsfaktor XIII durch Zugabe von Ethanol bis zu einer Endkonzentration der Lösung von 9 Vol.-% bei -3°C ausfällt, den Niederschlag bei einem pH-Wert von 6,35 und 37°C unter Rühren in einem Citratpuffer zu der gewünschten Fibrinogenkonzentration auflöst und die Lösung über eine Filterklärscheibe und ein Millipore-Sterilfilter filtriert.

2. Verfahren nach Anspruch 1, dadurch gekennzeichnet, daß die Behandlung mit β-Propiolacton in einem Konzentrationsbereich von 0,2 bis 0,35 Vol.-% bezogen auf das eingesetzte Plasma und bei einem pH-Wert von 7,2 erfolgt.

3. Verfahren nach Anspruch 1, dadurch gekennzeichnet, daß als Proteine adsorbierende Anionenaustauscher Diethylaminoethyl-Gruppen tragende, quervernetzte Dextrane oder Cellulose in einer Menge von 0,5 bis 3 g pro 1 kg Plasma eingesetzt werden.

4. Verfahren nach Anspruch 1, dadurch gekennzeichnet, daß die Fällung der Begleitproteine aus 1 kg Fibrinogenlösung mit 260 ml Ethanol, 240 ml Glycin-Citrat-Puffer und 1500 ml Natriumcitratlösung erfolgt.

5. Verfahren nach Anspruch 1, dadurch gekennzeichnet, daß die gewünschte Fibrinogenkonzentration in der Plasmaproteinlösung im Bereich von 40 bis 80 g/l liegt.